# EUROPEAN PATENT SPECIFICATION

(11) **EP 3 363 891 B1**
(45) Date of publication and mention of the grant of the patent: **08.04.2020**
(21) Application number: 17156525.2
(22) Date of filing: 16.02.2017
(51) Int. Cl.: C12N 1/14, C12R 1/68, C12P 21/02

(54) **PROCESS TO PRODUCE BIOMASS AND BIOGENIC SUBSTANCES UNDER SELECTIVE CONDITIONS**
VERFAHREN ZUR HERSTELLUNG VON BIOMASSE UND BIOGENEN SUBSTANZEN UNTER SELEKTIVEN BEDINGUNGEN
PROCÉDÉ DE PRODUCTION DE BIOMASSE ET DE SUBSTANCES BIOGÉNIQUES DANS DES CONDITIONS SÉLECTIVES

(43) Date of publication of application: 22.08.2018
(73) Proprietor: Brandenburgische Technische Universität Cottbus-Senftenberg, 01968 Senftenberg (DE); Universiti Kebangsaan Malaysia, Selangor (MY)
(72) Inventor: Ng, Chyan Leong, 57000 Kuala Lumpur (MY); Barig, Susann, 03238 Finsterwalde (DE); Stahmann, Klaus-Peter, 01994 Annahütte (DE)
(74) Representative: Simandi, Claus

(56) References cited:
- WO-A1-2008/067882
- SUSANN BARIG ET AL: "Monoseptic growth of fungal lipase producers under minimized sterile conditions: Cultivation of Phialemonium curvatum in 350?L scale", ENGINEERING IN LIFE SCIENCES, vol. 11, no. 4, 1 August 2011 (2011-08-01) , pages 387-394, XP055087408, ISSN: 1618-0240, DOI: 10.1002/elsc.201000219

## Description

The object of the invention is a process to produce biomass and biogenic substances obtainable by this process, wherein the microorganism refers to *Aspergillus fumigatus* AR04.

For approximately one hundred years the conscious cultivation of microorganisms for various applications such as feed and food as well as the transformation of organic materials to produce industrial-relevant metabolites or proteins (e.g. enzymes, antibodies) is used. Products are used in food industry, as pharmaceuticals, in chemical industry or as platform chemicals for further reactions. A prominent example is the industrial production of citric acid by *Aspergillus niger* which started in 1923. Milestones in medicine were the production of penicillin by *Penicillium chrysogenum* in 1942, the recombinant biosynthesis of an insulin precursor by *Escherichia coli* in 1982 (Hoechst AG) and most recently the production of artemisinic acid by *Saccharomyces cerevisiae.* Today hundreds of industrial-relevant products are generated with the help of bacteria and fungi. Mostly, the benefits of the production by microorganisms predominate over the chemical production process.

Microorganisms can also contribute to replace petroleum based precursors for chemical syntheses by re-growing natural products. A current example is the production of succinic acid. Succinic acid is ranked as one of the twelve most interesting products which should be produced by sugar-derived microbial conversion. It is the precursor for the production of several biodegradable plastics with an annual market of 30-50 kilotons/year. At the moment it is chemically produced from the petroleum derivative maleic anhydride (Jansen and Gulik, 2014).

For the production of bio-based products steel fermenters with large volumes of 100 to 500 m³ are needed. These fermenters need to be made of steel to allow heat sterilization. These facts indicate high investment costs for equipment and sterile technique. Furthermore, the running costs which include costs for medium, additives, energy and cooling are very high in such production plants contributing to the overall costs. To sum up, the calculation of costs already hinders bio-based production of for example basic chemicals, pharmaceuticals and enzymes.

Recently, a cultivation system was developed that lowers costs for investment and running. The main idea was to set up stringent cultivation conditions leading in monoseptic growth of one microorganism of interest. The conditions included low pH, room temperature, mineral salts without complex media components, plant oil as sole carbon and energy source, nitrate as nitrogen source and aerobic conditions. A plastic barrel instead of a steel fermenter lowers investment costs. Running costs are reduced by the mineral salts medium and the omission of sterilization. This cultivation system is called selective and minimal-sterile technique (Stahmann et al. 2008; Barig et al. 2011).

Industrial processes for the microbial production of food, pharmaceuticals or chemicals use established production strains. In the food sector mostly the experience and the traditional used strains play a role. To further drop costs for equipment and running of a microbial cultivation system the already established cultivation with selective and minimal-sterile technique as disclosed by Stahmann et al. (2008) is the ground for the present invention.

Hence, there is great need for improved manufacturing processes in order to produce biomass and related biogenic substances such as enzymes.

Sterile technology means not only high investment costs for autoclaves and steam generators as well as temperature-stable and pressure-resistant containers, pipelines and valves made of steel, but also high operating costs in form of energy as well as time for heating and cooling.

Because of these high costs, the space-time yields must be maximized by way of high growth rates and high final cell densities. Consequently, complex media with expensive constituents, e.g. yeast extract, are used first. In addition, for optimal gas exchange, stirrers must be operated at high speed. Moreover, in the case of recombinant methods, the complex media do not permit the use of complementation markers, e.g. for amino acid auxotrophies. Instead, for example for the production of recombinant enzymes with Bacillus spec., dominant markers, i.e. resistance genes for antibiotics, are used. The latter represent a further cost factor. In this case, it is a disadvantage that the use of antibiotics results in limitations, e.g. when the products are used for nutrition.

WO2008067882A1 refers to the monoseptic production of enzymes derived from biomass using fungus and at least one carbon source. Anyway, the space-time yields are not sufficient and shall be improved.

The object of the present invention is therefore based on providing a process for producing biomass and related biogenic substances, which overcomes the disadvantages summarized above.

Surprisingly, it was found that *A. fumigatus* AR04 allows a maximum growth rate in comparison to other microorganisms as indicated in Table 5 and Table 6.

Hence, such a growth rate over all temperatures allows the production of biomass in a short time and faster as all compared microorganism resulting advantageously in the maintenance of sterile condition within the biomass production time.

*A. fumigatus* AR04 was deposited at DSMZ (Leibniz Institute DSMZ-Deutsche Sammlung von Mikroorganismen und Zellkulturen GmbH) on 10.10.2016 having the Accession Number DSM 32373, hereinafter DSM.

In a preferred embodiment of the invention the carbon and energy source is a fatty oil, preferably one or more vegetable oil, like soya oil, sunflower oil or rapeseed oil.

In a very preferred embodiment of the invention the carbon and energy source is a vegetable oil, namely palm oil, cf. Table 1.1. and 1.2. It was found that best results can be achieved by using crude palm oil, pure palm oil or Malaysian palm oil.

Hence, the present invention relates to a culture process for producing biomass and related biogenic substances, comprising the steps of
a.) providing a minimal medium comprising water, mineral salts and an organic carbon source, in a vessel, wherein the organic carbon source is a fatty oil,
b.) inoculating the minimal medium with *A*. *fumigatus* AR04, in particular DSM 32373,
c.) obtaining the biomass,
d.) optionally, isolating the related biogenic substances like enzymes.

In a preferred embodiment, the minimal medium and the vessel are not sterilized.

In a further preferred embodiment of the invention the pH value is preferably less than 4, most preferably 1 to 3.

In a further preferred embodiment of the invention the temperature, in particular the incubation temperature is preferably less than 45 degrees Celsius, most preferably more than 30 degrees Celsius.

The term "biogenic substances" as used in this invention means that the inventive process allows the production of substances or chemical substances derived from biomass, preferably enzymes, proteins, nucleic acids, polysaccharides, lipids, vitamins, fine chemicals or others.

The chemical substances are preferably organic molecules, which can contain, in addition to carbon (C) and hydrogen (H), heteroatoms such as oxygen (O), nitrogen (N), sulfur (S), and phosphorus (P). The chemical substance can have linear and/or cyclic carbon chains, along with heteroatoms. It is preferable for the organic molecules to have less than 1,000 g/mol.

The biogenic substances can be isolated and purified from the organisms according to known processes. The resulting biogenic substances can be isolated by disruption of the cells or purification from the supernatant. The resulting products and compounds can be identified, e.g., by means of chromatography like LC/MS.

Essential for the invention is the isolation of a microorganism, which will reliably establish itself on a non-sterilised medium, namely *A. fumigatus* AR04, in particular DSM 32373 Concrete conditions were set as selective conditions as outlined within the present specification and examples.

In step b.), the fungus can initially settle visibly on the vessel wall as a biofilm. If the fungus is grown in suspension culture in a shake flask or baffled flask, the minimal medium becomes cloudy as a result of the fungus growth. This optical turbidity of the medium, which is visible to the naked eye, is also a sign that the fungus was grown for a sufficiently long period of time.

In step d), the biogenic substances can be obtained from the biofilm or from the minimal medium (culture broth).

It has been proven to be particularly advantageous to use nitrate as nitrogen source. Nitrate is inexpensive and excludes microorganisms as contaminants of the process, which cannot reduce nitrate.

In a preferred embodiment of the invention, the minimal medium comprises mineral salts, relative to one litre of water approximately 0.5 to 10 g KNO₃, approximately 0.5 to 5 g KH₂PO₄ approximately 0.2 to 0.75 g MgSO₄ X 7H₂O, approximately 0.2 to 0.75 g FeSO₄ x 7H₂O, approximately 0.2 to 0.75 g ZnSO₄ x 5H₂O, approximately 0.01 to 0.05 g CuSO₄ x 5H₂O, approximately 0.01 to 0.05 g MnCl₂ x 4H₂O and approximately 5 to 120 ml palm oil.

Most preferably, the minimal medium comprises mineral salts, relative to one litre of water approximately 1.5 g KNO₃, approximately 1.5 g KH₂PO₄, approximately 0.5 g MgSO₄ x 7H₂O, approximately 0.5 g FeSO₄ x 7H₂O, approximately 0.5 g ZnSO₄ x 5H₂O, approximately 0.02 g CuSO₄. x 5H₂O, approximately 0.02 g MnCl₂ x 4H₂O and approximately 20 ml palm oil.

In order, not to introduce any growth inhibiting substances via the water, deionised water is preferably used.

As biogenic substances production by the said fungus preferably takes place in an aerobic environment, in a further embodiment it is preferable to aerate the minimal medium. For this purpose, the aeration via a sterile filter and a frit is particularly suitable.

Even though the process can be carried out to the greatest extent under non-sterile conditions and thus extremely cost-effectively, it has proven advantageous to use sterile conditions for the inoculum culture with which the minimal medium is inoculated. This ensures that the minimal medium is inoculated with a pure fungus culture, which is not contaminated by bacteria or other undesirable microorganisms.

In order to simplify obtaining the biogenic substances from the process according to the invention, segments having a large surface and being easy to remove and easy to clean are introduced into the boiler or the large vessel, on which segments the fungus grows as biofilm and can subsequently be easily harvested from the culture and be further processed. Preferably, those funguses are hence used, in which the desired hydrolases are not freely diffusible but rather cell-associated.

The volume of the minimal medium is preferably approximately 1 liter to approximately 3,500 m³. Suitable is a volume of approximately 100 litres. A volume of approximately 350 litres has proven particularly suitable. In the simplest case, a large vessel or, alternatively, a boiler or a barrel which is inoculated with a spore suspension can be used as culture vessel.

The following examples and figures serve to explain the invention in detail, without, however, limiting the invention to these examples and figures.

### Examples and figures:

### Example 1:

### Palm oil is a suitable carbon source for cultivation under minimal sterile technique in Germany as well as in Malaysia

Palm oil is a natural carbon source with a unique composition and properties as presented in Table 1.1. Additional to triglycerides one can find free fatty acids, minerals and vitamins. In Table 1.2 the fatty acid composition of the triglyceride fraction is presented. Its unique combination of palmitic acid, oleic acid and further long chain fatty acids provides special properties which cannot be found in other natural oils. One important property is solidness at room temperature and it melts at 30-37 degrees Celsius. Furthermore, it is relatively stable towards heat degradation and oxidation. The special ingredients as well as the high productivity of the oil palm make the oil very interesting as carbon source for microbial growth.

**Table 1.1 Analytic data of the ingredients of crude palm oil (CPO). DOBI - Deterioration of bleachability index, Fe - Iron; Cu - Copper, P - Phosphate.**

| Parameter | | Crude palm oil Standard quality |
|---|---|---|
| Triglyceride | [%] | 96 |
| Free fatty acids | [%] | 3.5 |
| Moisture | [%] | 0.15 |
| Dirt | [%] | 0.02 |
| DOBI | [min] | 2.5 |
| Fe | [ppm] | < 5 |
| Cu | [ppm] | < 0.2 |
| P | [ppm] | < 20 |
| β-Carotene | [ppm] | 500 - 700 |

**Table 1.2 Fatty acid composition of the triglyceride fraction of crude palm oil**

| Fatty acid composition of the triglycerides | | Content [%] |
|---|---|---|
| Lauric acid | C12 : 0 | 0.14 |
| Myristic acid | C14 : 0 | 0.85 |
| Palmitic acid | C16 : 0 | 43 |
| Stearic acid | C18 : 0 | 4.3 |
| Oleic acid | C18 : 1 | 41 |
| Linoleic acid | C18 : 2 | 10 |
| Linolenic acid | C18 : 3 | 0.41 |
| Arachidonic acid | C20 : 0 | 0.35 |

### Malaysian palm oil is suitable as sole carbon and energy source for filamentous fungi in Germany

Two filamentous fungi were shown to grow on sterile solid media in Petri dishes using Malaysian palm oil as sole carbon and energy source. *Ashbya gossypii* is a well-known microorganism which is used for the industrial production of vitamin B2. Table 1.3 presents colony growth data on complex media containing yeast extract plus different carbon sources. For *A. gossypii* growth was observed on all media containing yeast extract and a carbon source such as glucose, rape seed oil or crude palm oil. Using mineral salts medium, no growth was observed independently from the carbon source. As second filamentous fungus Phialemonium curvatum AW02 was used. This microorganism was shown to grow under minimal-sterile conditions with rape seed oil as sole carbon and energy source (Stahmann et al., 2008; Barig et al., 2011). The subsequent table shows that exchanging the oil revealed similar colony growth rates. Interestingly, no difference between full media (with yeast extract) or minimal media was measured when comparing the different carbon sources.

**Table 1.3 Filamentous fungi show colony growth on Malaysian palm oil as sole carbon and energy source. Ashbya gossypii WT and Phialemonium curvatum AW02 were cultivated at their optimal growth temperatures (Tₒₚₜ) on agar plates containing different solid media. Radial growth was determined every 24 hours over 6 days and growth rates were calculated. Growth rates are rounded mean values of a minimum of three independent experiments. Yeast extract (YE); Glucose (G); Rape seed oil (RSO); Crude palm oil (CPO); Mineral salts medium (MSM).**

| Fungi | Tₒₚₜ [°C] | YE + G | YE+ RSO | YE+ CPO | MSM+ RSO | MSM+ CPO |
|---|---|---|---|---|---|---|
| | | Colony growth rate [µm/h] | | | | |
| *A. gossypii* WT | 28 | 230 | 92 | 150 | 0 | 0 |
| *P. curvatum* AW02 | 30 | 250 | 210 | 230 | 210 | 230 |

To demonstrate that Malaysian palm oil is also applicable under non-sterile conditions in a submerged culture 100-I-scale cultivations were carried out. *P. curvatum* AW02 was used as example microorganism in a 200 I plastic barrel. Running conditions were carried out as published in Barig et al., 2011. Table 1.4 summarizes the results. No contaminations were observed microscopically. An increase in temperature revealed higher yield in biomass and lipolytic activity. The higher cultivation temperature supported the growth of the fungal hyphae and, additionally, the liquid state of the palm oil which was than in turn better distributed in the culture broth.

**Table 1.4: Minimal-sterile cultivation in 100-L-scale was shown in Germany with crude palm oil from Malaysia. Growth of Phialemonium curvatum AW02 was carried out in 100 L minimal salts media containing plant oil as sole carbon and energy source. Cultivation was carried under selective, minimal-sterile conditions in a 200 I plastic barrel as published by Barig et al 2011. Each set of data represents a single but typical run.**

| | Rape seed oil | Crude palm oil | |
|---|---|---|---|
| Temperature [°C] | 19 | 19 | 30 |
| Contaminations | no | no | no |
| Final spore titer [cfu/ml] | 4 x 10⁶ | 7 x 10⁶ | 2 x 10⁷ |
| Wet biomass [g] | 330 | 130 | 750 |
| Total lipolytic activity [U] | 1,500 | 1,800 | 4,300 |
| Total cultivation time [d] | 8 | 7 | 7 |

In Malaysia, a tropical country, selective growth conditions allowing minimal-sterile cultivation technique worked with Malaysian palm oil.

If tested in field studies or in the laboratory, it is shown that the climate has an influence on the composition of the microflora. Temperature, humidity and also climate change factors such as the CO2 concentration are shown to might have an impact on complex changes in the overall community (Castro et al., 2010). Even if the investigated samples of the microflora are in the same country the influence of humid or continental climate has a large impact on the composition of the microbial community (Longo et al., 1991). Even if no publications exist which compare the microflora of Germany and Malaysia it can be assumed that the climatic differences lead to differences in the microbial composition in natural sources such as air, water and soil.

Therefore, the cultivation with minimal-sterile technique which was shown to run robust for more than 15 runs in Germany (Barig et al., 2011 and the current work) needed to be carried out in a tropic country. *P. curvatum AW02* as well as *Aspergillus niger,* a second robust filamentous fungus were cultivated under selective, minimal-sterile conditions using 2 L glass flasks and 200 L plastic barrels, respectively. The results are summarized in Table 1.5. The cultivations were carried out successfully concerning the requirement of zero contaminations. No other microorganisms could be detected either by microscopy or spreading a samples on full media agar plates. This shows that the cultivation technique under minimal-sterile conditions is not limited to Germany, a country of moderate climate. It is also applicable in areas of the world which show a different climate and therefore a different population of microorganisms in the natural sources which are used for cultivation e.g. water, air.

**Table 1.5: Minimal-sterile cultivation technique was shown in Malaysia with Malaysian crude palm oil in 0.5-L-scale and 100-L-scale. Cultivations of Phialemonium curvatum AW02 and Aspergillus niger WT were carried out in 0.5 L and 100 L minimal salts media, respectively. Crude palm oil was used as sole carbon and energy source. Cultivation was carried under minimal-sterile conditions in 2 L glass flasks or a 200-I plastic barrel, respectively as described in Barig et al., 2011. Contamination was tested both, by microscopy and cultivation of representative samples on agar plates.**

| **Strain** | ***Phialemonium curvatum* AW02** | ***Aspergillus niger* WT** |
|---|---|---|
| Cultivation Scale [L] | 0.5 | 100 |
| Temperature [°C] | 28 | 27 |
| Contaminations | No | No |
| Dry Biomass [g/L] | Not determined | 0.63 |
| Cultivation time [d] | 6 | 7 |

### Example 2:

Further filamentous fungi were found to accept stringent cultivation conditions in Malaysia and Germany.

A classical tool to find more microorganisms which are for example applicable in a specific cultivation system or produce an interesting metabolite is to screen for them in natural sources. In Germany and Malaysia a screening for filamentous fungi which are robust towards the stringent cultivation conditions of the minimal-sterile technique was carried out. The two additional criteria were the acceptance of crude palm oil as sole carbon and energy source and of temperatures above 30 °C. Table 2 summarizes the screened and identified strains from natural sources in Germany and Malaysia. Beside the already known strains for the cultivation under minimal-sterile technique it was possible to expand the existing list of applicable strains.

**Table 2: Thermotolerant fungi using crude palm oil as sole carbon and energy source were selected from different environments. Acidic mineral salts media (Barig et al., 2011) containing crude palm oil as carbon and energy source was used in Petri dish format. Soil samples were dispersed in 0.9 % NaCl and spread on the plates. Incubation of the plates was carried out at 30 °C, 40 °C or 50 °C. Growing microorganisms were separated. Identification of the isolates was carried out by sequencing of up to four genes (ITS = internal transcribed spacer, CaM = calmodulin, BenA = beta-tubulin and RPB2 = RNA polymerase 2) and subsequent comparison with the corresponding type strain sequences (Samson et al., 2011, 2014, Yilmaz et al., 2014) using IDNS database of SmartGene (version v3_6_14r2) and the CBS database (www.cbs.knaw.nl). Accession number in brackets.**

| Strain name | Environmental source [material; town; country] | Temperature during isolation process [°C] | sequence similarity with known species [%] | | | | |
|---|---|---|---|---|---|---|---|
| | | | ITS | CaM | BenA | RPB2 | type strain / nearest neighbor* |
| *Aspergillus fumigatus* AR01 | Soil; Senftenberg, Germany | 50 | 100 | | | | NRRL 163 |
| | | | (EF669931) | (EF669860) | (EF669791) | | |
| *Aspergillus fumigatus* AR04 | Plant compost; Wünschendorf, Germany | 50 | 100 | | | | NRRL 163 |
| | | | (EF669931) | (EF669860) | (EF669791) | | |
| *Thielavia terrestris* AR09 | Plant pot; Leipzig, Germany | 40 | 99 | n.d. | n.d. | n.d. | NRRL 8126* |
| | | | (CP003011) | | | | |
| *Aspergillus fumigatus* AR10 10 | Plant pot; Leipzig, Germany | 40 | 100 | | | | NRRL 163 |
| | | | (EF669931) | (EF669860) | (EF669791) | | |
| *Aspergillus asculeatus* L05 | Air exhaust of kitchen ventilation, Kampar, Malaysia | 40 | 99 | 96 | 96 | 98.6 | NRRL 5094 |
| | | | (EF661221) | (EF661148) | (HE577806) | (EF661046) | |
| *Penicillium oxalicum* L01 | Air exhaust of kitchen ventilation, Kampar, Malaysia | 30 | 100 | n.d. | n.d. | n.d. | NRRL 787 |
| | | | (NR121232) | | | | |
| *Talaromyces muroii* U05 | Air exhaust of kitchen ventilation, Kampar, Malaysia | 40 | 99 | n.d. | n.d. | n.d. | CBS 283.58 |
| | | | (KM066197) | | | | |
| *Aspergillus niger* U06 | Air exhaust of kitchen ventilation, Kampar, Malaysia | 30 | 100 | 98.4 | 99.6 | 98.1 | NRRL 326 |
| | | | (EF661186) | (EF661154) | (EF661089) | (EF661058) | |

### Example 3:

### A temperature of 40 °C is needed by Aspergillus fumigatus AR04 for maximal growth velocity

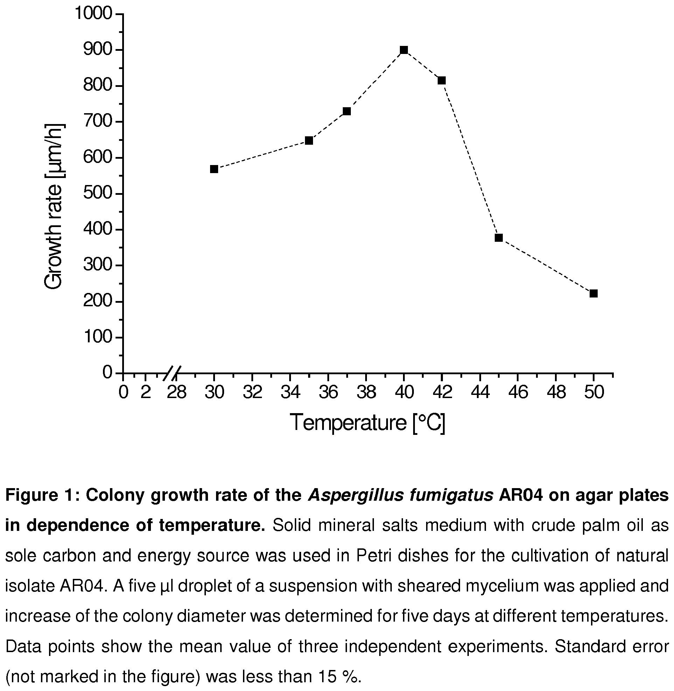

*Aspergillus fumigatus* AR04, one of the natural isolates identified and shown in Example 2, showed robustness towards high cultivation temperatures. Growth experiments were carried out on solid mineral salts medium with crude palm oil as sole carbon and energy source in Petri dishes. A suspension of fungal hyphae was applied in the center of each plate and the increase in diameter of the fungal colony was measured every day. The rate of colony increase was calculated and plotted against incubation temperature (cf. Figure 1). The robustness towards high temperature and all other conditions of the cultivation with minimal-sterile technique let become A. *fumigatus* AR04 an ideal example microorganism for the cultivation system.

### Example 4:

Growth rates of natural isolate *Aspergillus fumigatus* AR04 are 100 % higher on surface and in submerged culture in comparison with the industrial relevant fungus *Ashbya gossypii* ATCC 10895.

The next step was to show that the isolated and identified robust fungi can compete with an industrially relevant strain concerning growth rate that means biomass production, a sum parameter of metabolism. The growth rates of the natural isolate Aspergillus fumigatus AR04 and the wildtype of *Ashbya gossypii* ATCC 10895 were compared using two different methods. Firstly, solid mineral salts media containing crude palm oil and solid rich media were used as cultivation media. A suspension of fungal hyphae was centrally applied on the media and incubated at either 28 °C or 40 °C. 28 °C corresponds to the optimal growth temperature of *A. gossypii* ATCC 10895 thereby 40 °C corresponds to the optimal growth temperature of *A. fumigatus* AR04 (cf. Figure 1). Colony growth was observed daily and rates were calculated. Clearly, *A. fumigatus* AR04 showed higher growth rates than *A. gossypii* WT even at 28 °C or 40 °C on full media. Using mineral salts media *A. gossypii* WT showed no growth. This shows that the invention provides robust and thermophilic fungi which are able to compete with an industrial strain concerning growth.

**Table 3: Colony growth rates of Aspergillus fumigatus AR04 (DSM 32373) and Ashbya gossypii ATCC 10895 (WT). Solid mineral salts medium with crude palm oil (MSM) as sole carbon and energy source and solid rich medium (HA) were used in Petri dishes. Centrally five µl of a suspension with fungal cells was applied and hyphal growth was observed for seven days at two different temperatures. Increase in diameter over time was determined and growth rate was calculated. Mean values of three independent experiments are presented. Standard error was less than 20 %. "n.g." means no growth was observed.**

| | Growth rate [µm/h] | |
|---|---|---|
| | HA media | MSM |
| *Ashbya gossypii* WT | | |
| 28° C | 300 | n.g. |
| 40° C | 150 | n.g. |

| *Aspergillus fumigatus* AR04 | | |
|---|---|---|
| 28° C | 630 | 410 |
| 40° C | 1,100 | 710 |

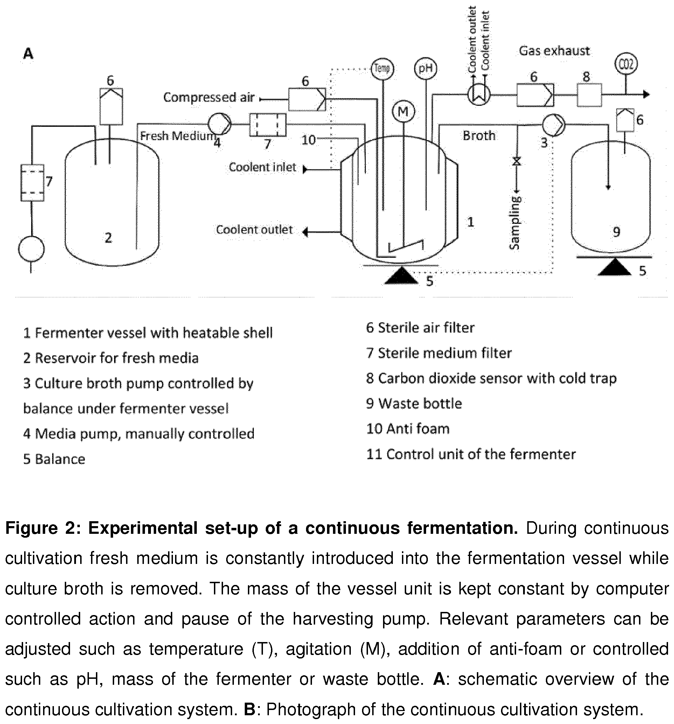

Only a small amount of processes does run as solid state fermentations. Most microbial industrial processes are performed in submerged cultures using steel fermenters in 100 cubic meters scale. Therefore, it is important to point out that the natural isolate can also compete in liquid medium with an industrial relevant strain. To reveal comparable data an established minimal medium for *Ashbya gossypii* (Stahmann, K. et al., 2001.) was used for continuous cultivation of *Aspergillus fumigatus* AR04. *A. fumigatus* AR04 was cultivated in a 7 L sterile fermenter under batch and continuous cultivation conditions. The general set-up of the experimental rig is disclosed in Figure 2. The following running conditions were applied: 3 L *A. gossypii* minimal medium, agitation 1,000 rpm, aeration 5 L/min with air, temperature 40 °C, antifoam 15 ml/h. The medium was sterile filtered and pumped into the fermenter until the volume of 3 L was reached in the fermenter. It was inoculated with 100 ml sheared A. fumigatus AR04 over-night pre-culture. For 4 hours the culture was run as batch. Afterwards, it was switched to continuous cultivation with a dilution rate (D) of 0.3 h-1 overnight. Continuously, fresh media was pumped in and simultaneously culture broth was pumped out. The harvesting pump was controlled by a balance which was placed under the fermenter. Next morning, the dilution rate was either kept constant or increased to D= 0.5 h-1 or D= 0.7 h-1 to analyze if A. fumigatus AR04 can grow against stepwise increasing dilution rates. After four residence times the first sample was taken determining dry biomass, CO2 in the exhaust and glucose in the culture filtrate. With intervals of one hour this sample collection was repeated two times. Table 4 summarizes the determined data. Constant biomass, CO2 concentration and glucose concentration in the culture broth indicate a steady-state situation of the culture. This results were compared with previous continuous cultivation experiments of *A. gossypii* ATCC 10895 under similar conditions. Differences were the growth temperature (28 °C for *A. gossypii* WT) and the agitation rate of 550 rpm. In a continuous cultivation the dilution rate equals to the growth rate of the microorganism. If the dilution rate is set above the maximal growth rate the biomass is washed out. This is a standard method to determine maximal growth rates. Furthermore, increasing dilution rates reveal decreasing biomass concentrations. Table 4 shows three different dilution rates which were applied to a continuous culture of A. fumigatus AR04. At a dilution rate of 0.3 h-1 Aspergillus fumigatus AR04 showed constant biomass production of approximately 4 g/l (dry weight). *A. gossypii* WT showed at D= 0.32 h-1 a biomass concentration of 0.91 g/l (Stahmann et al., 2001). That shows that *A. gossypii* WT is at D= 0.32 h-1 closer to its maximal growth rate than A. fumigatus AR04 (Stahmann et al., 2001). A. fumigatus AR04 showed still at 0.7 h⁻¹ biomass concentrations of approximately 1.2 g/l. Concluding to that data the maximal growth rate of the natural isolate A. fumigatus AR04 (DSM 32373) is higher than 0.7 h-1 (µmax> 0.7 h⁻¹).

Consistently, *A. fumigatus* AR04 (DSM 32373) has a higher growth rate on solid and in liquid media compared with the industrial relevant microorganism *A. gossypii* ATCC 10895.

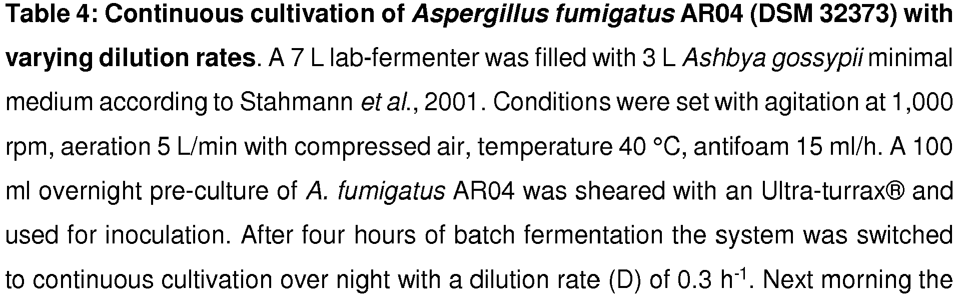

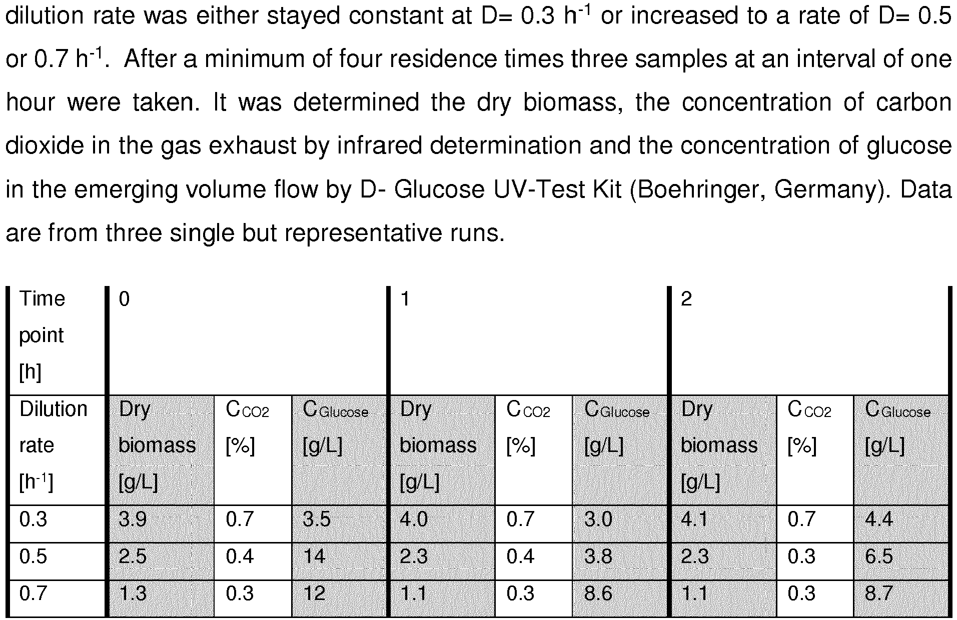

### Example 5:

**Table 5: Growth rates of different Aspergillus species and strains on agar plates containing mineral salts medium with crude palm oil as sole source of carbon and energy. Mean values were obtained from two or three independent experiments, as scattering indicator standard error was calculated; n.g. means no growth.**

| ***Aspergillus* species** | **Growth rate [µm h⁻¹] at** | | | | | |
|---|---|---|---|---|---|---|
| | **28°C** | **31°C** | **34°C** | **37°C** | **40°C** | **43°C** |
| *A. niger* DSM 11167 | 470 ± 10 | 560 ± 10 | 650 ± 0 | 650 ± 30 | 540 ± 10 | n. g. |
| *A. oryzae* DSM 63303 | 310 ± 10 | 490 ± 10 | 330 ± 10 | 310 ± 20 | 260 ± 10 | n. g. |
| **A. fumigatus AR04 (DSM 32373)** | **510 ± 10** | **620 ± 10** | **760 ± 20** | **830 ± 20** | **840 ± 10** | **630 ± 80** |
| *A. fumigatus* ATCC 46645 | 440 ± 20 | 580 ± 20 | 730 ± 0 | 760 ± 10 | 670 ± 30 | 470 ± 10 |
| *A. fumigatus* Af 293 (CBS) | 440 ± 10 | 530 ± 10 | 610 ± 10 | 520 ± 40 | 630 ± 0 | 510 ± 30 |

### Conclusion:

Our natural isolate A. *fumigatus* AR04 (DSM 32373) refer to the best performances of growth rates at all temperatures.

### Example 6:

**Table 6: Growth rates of filamentous fungi reported in literature. Maximal rates measured as biomass increase in submerged cultures. Industrially relevant, that means applied in thousand tons per year scale are Aspergillus niger (citric acid), Aspergillus oryzae (food fermentations), Ashbya gossypii (riboflavin) and Fusarium venenatum (Mycoprotein). Aspergillus fumigatus, Aspergillus nidulans and Thermomyces lanuginosus are listed because they were studied at higher temperatures.**

| **Species** | **Cultivation type** | **Specific growth rate** | | **Reference** |
|---|---|---|---|---|
| | | **at** | **[h⁻¹]** | |
| **Applied** | | | | |
| *Aspergillus niger* | Batch, stirred tank reactor | 30°C | 0.29 | Jorgensen *et al.* 2007 |
| *Aspergillus niger* | Batch, stirred tank reactor | 30°C | 0.26 | Lameiras *et al.* 2015 |
| *Aspergillus niger* | Batch, shake flasks | 30°C | 0.24 | Rajasekaran and Maheshwari 1990 |
| *Aspergillus oryzae* | Batch, stirred tank reactor | 30°C | 0.27 | Carlsen *et al.* 1996a |
| *Aspergillus oryzae* | Chemostat | 30°C | 0.17 | Carlsen *et al.* 1996b |
| *Ashbya gossypii* | Chemostat | 28°C | 0.32 | Stahmann *et al.* 2001 |
| *Fusarium venenatum* | Batch, shake flasks | 25°C | 0.21 | Wiebe *et al.* 2000 |
| | Chemostat | 25°C | 0.17 | |

| **Thermophilic** | | | | |
|---|---|---|---|---|
| *Thermomyces lanuginosus* | Batch, shake flasks | 50°C | 0.23 | Rajasekaran and |
| | | 30°C | 0.06 | Maheshwari 1990 |
| *Aspergillus nidulans* | Batch, shake flasks | 30°C | 0.22 | Trinci 1969 |
| | | 37°C | 0.36 | |
| *Aspergillus fumigatus* | Chemostat | 37°C | 0.25 | Vödisch *et al.* 2011 |

### Literature:

Barig S., Alisch R., Nieland S., Wuttke A., Gräser Y., Huddar M., Schnitzlein K., Stahmann K.-P. (2011). Monoseptic growth of fungal lipase producers under minimized sterile conditions: Cultivation of Phialemonium curvatum in 350 L scale. Engeneering in Life Sciences, 11, 387-394.
Carlsen, M., Spohr, A. B., Nielsen, J., & Villadsen, J. (1996a). Morphology and physiology of an α-amylase producing strain of Aspergillus oryzae during batch cultivations. Biotechnology and Bioengineering, 49(3), 266-276.
Carlsen, M., Nielsen, J., & Villadsen, J. (1996b). Growth and α-amylase production by Aspergillus oryzae during continuous cultivations. Journal of Biotechnology, 45(1), 81-93.
Castro H.F., Classen A.T., Austin E., Norby R.J. and Schadt C.F. (2010). Microbial Community Responses to Multiple Experimental Climate Change Drivers. Applied and Environmental Microbiology, 70(4), 999-1007.
Jansen M.L.A. and Gulik W.M. (2014). Towards large scale fermentative production of succinic acid. Current Opinion in Biotechnology, 30:190-197.
Jorgensen, T. R., Poulsen, B. R., Ruijter, G. J., Visser, J., & Iversen, J. J. (2007). Glucose uptake and growth of glucose-limited chemostat cultures of Aspergillus niger and a disruptant lacking MstA, a high-affinity glucose transporter. Microbiology, 153(6), 1963-1973.
Lameiras, F., Heijnen, J. J., & van Gulik, W. M. (2015). Development of tools for quantitative intracellular metabolomics of Aspergillus niger chemostat cultures. Metabolomics, 11(5), 1253-1264.
Longo E., Cansado J., Agrelo D., Villa T.G. (1991). Effect of Climatic Conditions on Yeast Diversity in Grape Musts from Northwest Spain. Am. J. Enol. Vitic, 42(2), 141-144.
Rajasekaran, A. K., & Maheshwari, R. (1990). Growth-Kinetics and Intracellular Protein Breakdown in Mesophilic and Thermophilic Fungi. Indian Journal of Experimental Biology, 28(1), 46-51.
Samson R.A., Visagie C.M., Houbraken J., Hong S.-B., Hubka V., Klaassen C.H.W, Perrone G., Seifert K.A., Susca A., Tanney J.B., Varga J., Kocsubé S., Szigeti G., Yaguchi T., Frisvad J.C. (2014). Phylogeny, identification and nomenclature of the genus Aspergillus. Studies in Mycology, 78, 141-173.
Samson R.A., Yilmaz N., Houbraken J., Spierenburg H., Seifert K.A., Peterson S.W., Varga J. & Frisvad J.C. (2011). Phylogeny and nomenclature of the genus Talaromyces and taxa accommodated in Penicillium subgenus Biverticillium. Studies in Mycology, 70, 159-183.
Stahmann, K., Arst, H. N., Althöfer, H., Revuelta, J. L., Monschau, N., Schlüpen, C., & Schlösser, T. (2001). Riboflavin, overproduced during sporulation of Ashbya gossypii, protects its hyaline spores against ultraviolet light. Environmental microbiology, 3(9), 545-550.
Stahmann K.-P., Nieland S., Wuttke A. (2008). Mikrobielles Verfahren zur Herstellung von Enzymen. Patent DE 10 2006 057 724 A1.
Trinci, A. P. J. (1969). A kinetic study of the growth of Aspergillus nidulans and other fungi. Microbiology, 57(1), 11-24.
Vödisch, M., Scherlach, K., Winkler, R., Hertweck, C., Braun, H. P., Roth, M., Kniemeyer, O. (2011). Analysis of the Aspergillus fumigatus proteome reveals metabolic changes and the activation of the pseurotin A biosynthesis gene cluster in response to hypoxia. Journal of proteome research, 10(5), 2508-2524.
Wiebe, M.G., Robson, G.D., Shuster, J., Trinci A.P.J. (2000). Evolution of a Recombinant (Gucoamylase-Producing) Strain of Fusarium venenatum A3/5 in Chemostat Culture. BIOTECHNOLOGY AND BIOENGINEERING, 73(2), 146-156.
Yilmaz N., Visagie C.M., Houbraken J., Frisvad J.C., Samson R.A. (2014). Polyphasic taxonomy of the genus Talaromyces. Studies in Mycology, 78: 175-341.

*The inventors are grateful to SIME DARBY TECHNOLOGY CENTER (Malaysia) for providing with palm oil and analytical data.*

## Claims

1. A culture process for producing biomass and related biogenic substances, comprising the steps of:
a.) providing a minimal medium comprising water, mineral salts and an organic carbon source in a vessel, wherein the organic carbon source is a fatty oil,
b.) inoculating the minimal medium with *A. fumigatus* AR04, DSM 32373,
c.) obtaining the biomass,
d.) optionally, isolating the related biogenic substances like enzymes.

2. The culture process according to claim 1, wherein the carbon source is a fatty oil selected from a vegetable oil, in particularsoya oil, sunflower oil or rapeseed oil.

3. The culture process according to claim 1, wherein the carbon source is a fatty oil selected from palm oil, in particular crude palm oil, pure palm oil or Malaysian palm oil.

4. The process according to one of the preceding claims, wherein the temperature is less than 45 degrees Celsius or more than 30 degrees Celsius.

5. The process according to one of the preceding claims, wherein the pH value is less than 4.

6. The process according to one of the preceding claims, wherein the minimal medium further compromises a nitrogen source, and the nitrogen source is nitrate.

7. A. fumigatus AR04 DSM 32373

## Patentansprüche

1. Kulturverfahren zur Herstellung von Biomasse und zugehörigen biogenen Substanzen umfassend die Schritte:
a.) Bereitstellen eines Minimalmediums enthaltend Wasser, Mineralsalze und einer organischen Kohlenstoffquelle in einem Behälter, wobei die organische Kohlenstoffquelle ein Fettöl ist,
b.) Beimpfen des Minimalmediums mit *A. fumigatus* AR04 DSM 32373,
c.) Erhalten der Biomasse,
d.) optional, Isolierung der zugehörigen biogenen Substanzen, wie Enzyme.

2. Kulturverfahren gemäß Anspruch 1, wobei die Kohlenstoffquelle ein Fettöl ist, ausgewählt aus einem Pflanzenöl, insbesondere Sojaöl, Sonnenblumenöl oder Rapsöl.

3. Kulturverfahren gemäß Anspruch 1, wobei die Kohlenstoffquelle ein Fettöl ist, ausgewählt aus Palmöl, insbesondere ein Rohpalmöl, reines Palmöl oder malaysisches Palmöl.

4. Verfahren nach einem der vorstehenden Ansprüche, wobei die Temperatur weniger als 45 °C oder mehr als 30 °C ist.

5. Verfahren nach einem der vorstehenden Ansprüche, wobei der pH-Wert weniger als 4 ist.

6. Verfahren nach einem der vorstehenden Ansprüche, wobei das Minimalmedium weiter enthält eine Stickstoffquelle und die Stickstoffquelle Nitrat ist.

7. A fumigatus AR04 DSM 32373.

## Revendications

1. Procédé de culture pour produire une biomasse et des substances biogéniques apparentées, comprenant les étapes :
a) d'alimentation en un milieu minimal comprenant de l'eau, des sels minéraux et une source de carbone organique dans un récipient, où la source de carbone organique est une huile grasse,
b) d'inoculation de *A. fumigatus* AR04, de DSM 32373 dans le milieu minimal,
c) d'obtention de la biomasse,
d) éventuellement, de séparation des substances biogéniques apparentées comme des enzymes.

2. Procédé de culture selon la revendication 1, dans lequel la source de carbone est une huile grasse choisie parmi une huile végétale, en particulier, l'huile de soja, l'huile de tournesol ou l'huile de colza.

3. Procédé de culture selon la revendication 1, dans lequel la source de carbone est une huile grasse choisie parmi l'huile de palme, en particulier, l'huile de palme brute, l'huile de palme pure ou l'huile de palme de Malaisie.

4. Procédé selon l'une des revendications précédentes, dans lequel la température est inférieure à 45 degrés Celsius ou supérieure à 30 degrés Celsius.

5. Procédé selon l'une des revendications précédentes, dans lequel la valeur du pH est inférieure à 4.

6. Procédé selon l'une des revendications précédentes, dans lequel le milieu minimal comprend en outre une source d'azote, et la source d'azote est un nitrate.

7. A. fumigatus AR04 de DSM 32373.
